(19) Europäisches Patentamt
European Patent Office
Office européen des brevets

(11) Veröffentlichungsnummer: **0 101 806**
**B1**

(12) **EUROPÄISCHE PATENTSCHRIFT**

(45) Veröffentlichungstag der Patentschrift: **06.08.86**

(51) Int. Cl.⁴: **C 07 C 143/58,** C 07 C 139/00

(21) Anmeldenummer: **83105897.9**

(22) Anmeldetag: **16.06.83**

(54) **Verfahren zur Herstellung von 1-Aminobenzol-2-sulfonsäuren.**

(30) Priorität: **29.06.82 DE 3224155**

(43) Veröffentlichungstag der Anmeldung: **07.03.84 Patentblatt 84/10**

(45) Bekanntmachung des Hinweises auf die Patenterteilung: **06.08.86 Patentblatt 86/32**

(84) Benannte Vertragsstaaten: **CH DE FR GB LI**

(56) Entgegenhaltungen:
**EP - A - 0 025 274**
**US - A - 2 642 458**
**US - A - 3 038 932**
**US - A - 3 329 708**
**US - A - 3 670 002**

(73) Patentinhaber: **BAYER AG, Konzernverwaltung RP Patentabteilung, D-5090 Leverkusen 1 Bayerwerk (DE)**

(72) Erfinder: **Michna, Martin, Dr., Silesiusstrasse 74, D-5000 Köln 80 (DE)**
Erfinder: **Henk, Hermann, Dr., Roggendorfstrasse 55, D-5000 Köln 80 (DE)**

**EP 0 101 806 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Gegenstand der vorliegenden Erfindung ist ein Verfahren zur Herstellung von 1-Aminobenzol-2-sulfonsäuren der Formel

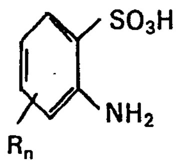

(I)

worin
R= H oder Substituent
n= 1–4
und deren Salzen, insbesondere den Alkalisalzen durch Oxidation von Verbindungen der Formel

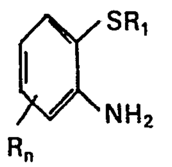

(II)

worin
$R_1$ = H, Kation, insbesondere Alkalimetallkation oder den Rest der Formel

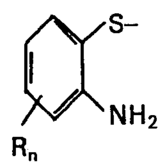

dadurch gekennzeichnet, dass man die Oxidation bei pH-Werten von 8–14 durchführt.

Die Verbindungen II erhält man beispielsweise durch alkalische Hydrolyse von Benzthiazolen der Formel

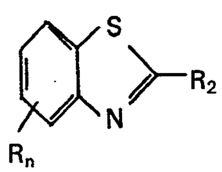

(III)

worin
$R_2$ = H, Substituent und
R und n die oben angegebene Bedeutung haben.

Die Oxidation erfolgt vorzugsweise in wässrigem Medium, das, falls erforderlich, gegen Oxidationsmittel unter den Reaktionsbedingungen stabile organische, wassermischbare Lösungsmittel enthalten kann.

Die Oxidation erfolgt bei Temperaturen von etwa 20 bis 100 °C, insbesondere etwa 50 bis 100 °C. Als Oxidationsmittel kommen beispielsweise in Frage: Sauerstoff, Halogene wie Chlor oder Brom, Permanganate, Chromate, Nitrate, Eisen-III-salze und bevorzugt Peroxide, insbesondere Wasserstoffperoxid sowie andere Perverbindungen wie Perborate, Persulfate, Percarbonate, Percarbonsäuren und Persulfonsäuren. Gegebenenfalls können auch geeignete Katalysatoren zugesetzt werden, beispielsweise Wolframate, Vanadate, Molybdate und Eisen-II-salze.

Die Oxidationsmittel werden in Mengen von etwa 1–6 Mol pro Mol II eingesetzt.

Die Substituenten R können gleich oder verschieden sein. Geeignete Substituenten sind beispielsweise Alkyl, insbesondere gegebenenfalls substituiertes $C_1$–$C_4$-Alkyl, Aryl, insbesondere gegebenenfalls substituiertes Phenyl, Nitro, Sulfo, CN, Halogen, insbesondere Cl, OH, SH, $NH_2$, Alkoxy, insbesondere gegebenenfalls substituiertes $C_1$–$C_4$-Alkoxy, Arylazo insbesondere gegebenenfalls substituiertes Phenylazo, gegebenenfalls weiter substituiertes Benztriazolyl-2 oder Naphthtriazolyl-2.

Geeignete Substituenten $R_2$ sind insbesondere $NH_2$, OH, Cl, $SO_3H$, SH, Alkyl, insbesondere ggf. substituiertes $C_1$–$C_4$-Alkyl.

Geeignete Ausgangsverbindungen III sind beispielsweise Benzthiazol, Mercaptobenzthiazol, 2-Methyl-6-nitrobenzthiazol, 2-Amino-6-methoxy-benzthiazol, 2-Chlorbenzthiazol, 6-Sulfobenzthiazol.

Eine bevorzugte Ausgestaltung des erfindungsgemässen Verfahrens besteht darin, dass man die Verbindungen III alkalisch hydrolysiert, vorzugsweise bei pH-Werten von etwa 10 bis 14 und zwar in wässrigem oder wässrigorganischem Medium und anschliessend ohne Zwischenisolierung bei pH-Werten von etwa 8–14 oxydiert, vorzugsweise mit Wasserstoffperoxid.

Die Reaktion kann auch so durchgeführt werden, dass gleichzeitig verseift und zu I oxidiert wird. In einer weiteren Verfahrensvariante kann auch zunächst III verseift, dann mit Oxidationsmitteln unter milden Bedingungen zum Disulfid oxidiert und dieses anschliessend unter schärferen Bedingungen zu I oxidiert werden.

Die Verbindungen I sind bekannt. Sie werden beispielsweise als Diazokomponenten für eine Herstellung von Azofarbstoffen eingesetzt. Es war bereits aus der US-Patentschrift 30 38 932 bekannt, I aus II durch Oxidation in stark saurem Medium herzustellen.

Überraschenderweise lässt sich die Oxidation auch in alkalischem Medium durchführen, ohne dass Nebenprodukte auftreten, man erhält vielmehr reinere Produkte als bei der Oxidation im stark sauren Medium.

Beispiel 1

35 g Mercaptobenzthiazol werden mit 50 g NaOH und 400 ml Wasser 3 bis 5 Stunden am Rückfluss gekocht. Die erhaltene Lösung lässt man auf 50 °C abkühlen und tropft langsam so viel 30%iges Wasserstoffperoxid zu, bis kein weiteres Oxidationsmittel mehr verbraucht wird. Man erhitzt noch kurz auf 80–90 °C, gibt ca. 10 g A-Kohle und etwas Filtrierhilfsmittel zu und filtriert unlösliche Rückstände ab. Dann lässt man abkühlen, fällt die gebildete o-Sulfanilsäure durch Ansäuern auf pH 1 aus, saugt ab und trocknet.

Ausbeute ca. 30–31 g.

Beispiel 2

1 Mol 2-Methyl-6-nitro-benzthiazol werden in 1,2 l 3%iger NaOH so lange gekocht bis eine Lösung vorliegt. Diese lässt man abkühlen auf ca. 30 °C und tropft in ca. 1 Std. 1 Mol Wasserstoffperoxid als 35%ige wässrige Lösung zu. Die Temperatur sollte nicht über 50 °C steigen. Anschliessend tropft man in 1 Std. weitere 2 Mol 35%iges Wasserstoffperoxid zu, wobei die Temperatur bis zum Siedepunkt steigt. Man lässt auf 80 °C abkühlen, setzt 10 g A-Kohle und 10 g eines üblichen Filtrierhilfsmittels zu, filtriert und kühlt auf 5–10 °C ab und fällt durch Zugabe von konzentrierter Salzsäure die gebildete 2-Amino-5-nitro-benzolsulfonsäure aus.

Ausbeute ca. 80 % der Theorie.

Beispiel 3

18 g 2-Amino-6-methoxy-benzthiazol werden mit 300 ml Wasser und 15 g NaOH gekocht bis kein Ammoniak mehr entweicht. Die erhaltene Lösung wird auf 50 °C gebracht und so lange Luft durchgeleitet bis ein Chromatogramm die vollständige Oxidation zum 2,2'-Diamino-5,5'-dimethoxydiphenyldisulfid anzeigt. Anschliessend werden 18 ml 35%iges Wasserstoffperoxid zugetropft, wobei die Temperatur auf 80–100 °C steigt. Man setzt 10 g Aktivkohle und 5 g Kieselgur zu, verrührt 30 Minuten und klärt. Die erhaltene Lösung des Natriumsalzes der 2-Amino-5-methoxybenzolsulfonsäure kann direkt weiter verarbeitet werden.

Beispiel 4

1 Mol Benzthiazol wird mit 200 ml Wasser und 220 ml 40%iger Natronlauge unter kräftigem Rühren 5 Stunden am Rückfluss gekocht. Die Lösung wird auf 20–30 °C abgekühlt. Zur abgekühlten Lösung gibt man langsam zunächst 86 ml 35%iges Wasserstoffperoxid. Dabei soll die Temperatur 50 °C nicht überschreiten. Anschliessend werden im Verlauf von einer Stunde weitere 152 ml 35%iges Wasserstoffperoxid zugetropft. Die Temperatur steigt dabei auf 95 °C und das zunächst ausgefallene 2,2'-Diaminodiphenyldisulfid geht in 2-Aminobenzolsulfonat über. Nach Ende der Wasserstoffperoxidzugabe wird noch 30 Minuten bei 95 °C gehalten. Man lässt auf 80 °C abkühlen, setzt 5 g Aktivkohle und 10 g übliches Filtrierhilfsmittel zu und klärt. Die geklärte Lösung wird auf 5–10 °C gekühlt, auf pH 0,5–1 angesäuert und die ausgefallene o-Sulfanilsäure abgesaugt. Ausbeute: 122 g (70%). Die Mutterlauge enthält weitere 30 g o-Sulfanilsäure.

**Patentansprüche**

1. Verfahren zur Herstellung von 1-Aminobenzol-2-sulfonsäuren der Formel

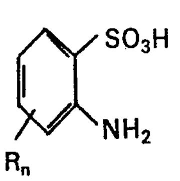

worin

R = H oder Substituent,

n = 1–4

und deren Salzen, insbesondere deren Alkalisalze durch Oxidation von Verbindungen der Formel

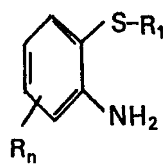

worin

$R_1$ = H, Kation, insbesondere Alkalimetallkation oder Rest der Formel

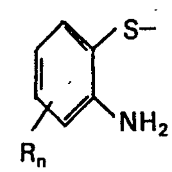

dadurch gekennzeichnet, dass man die Oxidation bei pH-Werten von 8–14 durchführt.

2. Verfahren nach Anspruch 1, dadurch gekennzeichnet, dass man in wässrigem Medium oxidiert.

3. Verfahren nach Ansprüchen 1–2, dadurch gekennzeichnet, dass man bei Temperaturen von etwa 50–100 °C oxidiert.

4. Verfahren nach Ansprüchen 1–3, dadurch gekennzeichnet, dass man mit Wasserstoffperoxid oxidiert.

5. Verfahren zur Herstellung der 1-Aminobenzolsulfonsäuren des Anspruchs 1, dadurch gekennzeichnet, dass man Benzthiazole der Formel

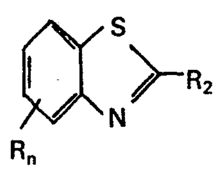

worin

R und n die in Anspruch 1 angegebene Bedeutung haben und

$R_2$ für H oder einen Substituenten steht, in wässrigem oder wässrig-organischen Medium hydrolysiert und die dabei erhaltenen Reaktionsprodukte ohne Zwischenisolierung bei pH-Werten von 8–14 oxidiert.

**Revendications**

1. Procédé de production d'acides 1-aminobenzène-2-sulfoniques de formule:

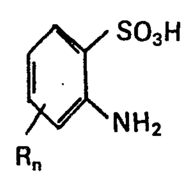

dans laquelle

R représente un atome d'hydrogène ou un substituant, et

n vaut 1–4,

et de leurs sels, en particuliers leurs sels de métaux alcalins, par oxydation de composés de formule:

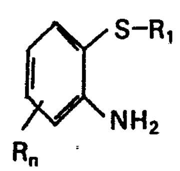

dans laquelle

$R_1$ représente un atome d'hydrogène, un cation, notamment un cation de métal alcalin, ou le reste de formule:

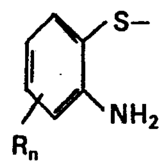

procédé caractérisé en ce qu'on conduit l'oxydation à des pH de 8 à 14.

2. Procédé selon la revendication 1, caractérisé en ce qu'on oxyde en milieu aqueux.

3. Procédé selon les revendications 1, 2, caractérisé en ce qu'on oxyde à des températures d'environ 50 à 100 °C.

4. Procédé selon les revendications 1 à 3, caractérisé en ce qu'on oxyde à l'aide de peroxyde d'hydrogène.

5. Procédé pour produire des acides 1-amino-benzène-sulfoniques selon la revendication 1, caractérisé en ce qu'on hydrolyse des benzothiazoles de formule:

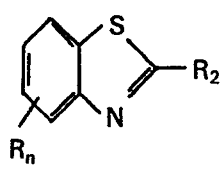

dans laquelle

R et n ont le sens indiqué à la revendication 1, et

$R_2$ représente un atome d'hydrogène ou un substituant, en milieu aqueux ou hydro-organique, l'on oxyde à des pH de 8 à 14, sans isolement intermédiaire, les produits de réaction ainsi obtenus.

## Claims

1. Process for the preparation of 1-aminobenzene-2-sulphonic acids of the formula

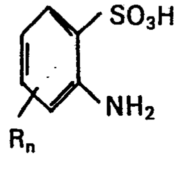

wherein

R = H or a substituent and

n = 1–4,

and their salts, in particular their alkali metal salts, by oxidation of compounds of the formula

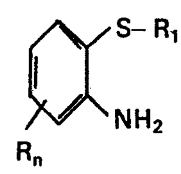

wherein

$R_1$ = H, a cation, in particular an alkali metal cation, or a radical of the formula

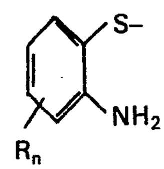

characterised in that the oxidation is carried out at pH values of 8–14.

2. Process according to Claim 1, characterised in that oxidation is effected in an aqueous medium.

3. Process according to Claims 1–2, characterised in that oxidation is effected at temperatures of about 50–100 °C.

4. Process according to Claims 1–3, characterised in that oxidation is effected with hydrogen peroxide.

5. Process for the preparation of the 1-aminobenzene-sulphonic acids of Claims 1, characterised in that benzothiazoles of the formula

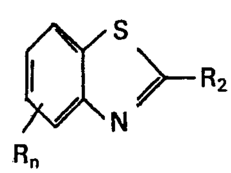

wherein

R and n have the meaning given in Claim 1 and

$R_2$ represents H or a substituent,

are hydrolysed in an aqueous or aqueous-organic medium, and the resulting reaction products, without intermediate isolation, are oxidised at pH values of 8–14.